# EUROPEAN PATENT SPECIFICATION

(11) **EP 2 078 755 B1**
(45) Date of publication and mention of the grant of the patent: **20.02.2019**
(21) Application number: 07398019.5
(22) Date of filing: 18.12.2007
(51) Int. Cl.: C12P 1/02

(54) **Process for simultaneous extraction and purification of fine chemicals from spent mushroom compost, mushroom stems and partially degraded mushroom fruiting bodies**
Prozess zur simultanen Extraktion und Reinigung von Feinchemikalien aus verbrauchtem Pilzkompost, Pilzstämmen und teilweise abgebauten Pilzfruchtkörpern
Processus d'extraction simultanée et de purification de produits chimiques fins à partir d'un compost de champignon, pieds de champignon et corps de fructification de champignon partiellement dégradés

(43) Date of publication of application: 15.07.2009
(73) Proprietor: Karmali, Amin, 2785-300 S. Domingo de Rana (PT)
(72) Inventor: Karmali, Amin, 2785-300 S. Domingo de Rana (PT)

(56) References cited:
- WO-A-2006/133708
- WO-A-2007/018095
- DE-A1- 10 045 961
- WASSER S P: "Medicinal mushrooms as a source of antitumor and immunomodulating polysaccharidess" APPLIED MICROBIOLOGY AND BIOTECHNOLOGY, SPRINGER VERLAG, BERLIN, DE, vol. 60, 10 September 2002 (2002-09-10), pages 258-274, XP002402437 ISSN: 0175-7598
- GARTZ JOCHEN: "Extraction and analysis of indole derivatives from fungal biomass" JOURNAL OF BASIC MICROBIOLOGY, vol. 34, no. 1, 1994, pages 17-22, XP002484501 ISSN: 0233-111X
- SINGH A D ET AL: "OPTIMIZATION OF EXTRACTION OF BULK ENZYMES FROM SPENT MUSHROOM COMPOST" JOURNAL OF CHEMICAL TECHNOLOGY AND BIOTECHNOLOGY, BLACKWELL SCIENTIFIC PUBLICATIONS. OXFORD, GB, vol. 78, no. 7, 1 July 2003 (2003-07-01), pages 743-752, XP001177158 ISSN: 0268-2575
- BALL ANDREW S ET AL: "The recovery of lignocellulose-degrading enzymes from spent mushroom compost" BIORESOURCE TECHNOLOGY, vol. 54, no. 3, 1995, pages 311-314, XP002484502 ISSN: 0960-8524

## Description

### FIELD OF THE INVENTION

This invention relates to a process of simultaneous extraction and purification of enzymes, protein-bound polysaccharides, lectins, terpenes, alkaloids and antibiotics from either spent mushroom compost, mushroom stems or partially degraded mushroom fruiting bodies.

### BACKGROUND OF THE INVENTION

During the last decades, there has been a strong concern regarding the environmental pollution due to large quantities of agro-industrial by-products that are disposed in the environment. However, these agro-industrial by-products such as lignocellulosic wastes, corn cobs, sugar cane bagasse and coffee pulp can be used as low-cost carbohydrate sources for growth of fungi for production of suitable fine chemicals for food, textile, detergent and pharmaceutical industries. Fungal solid-state fermentation technology has been used for centuries for bioconversion of agro-industrial wastes by using basidiomycete strains, namely in mushroom production.

The mushroom growing industry generates about 200 million ton/year of spent mushroom compost which also contains mushroom stems and partially degraded mushroom fruiting bodies. Since this huge waste material is not suitable to be re-utilized in mushroom production, it is either used as garden fertilizer or deposited in landfill which results in environmental pollution and public health problems according to several reports from World Health Organization (WHO). Therefore, the present invention involves a novel process for simultaneous extraction and purification of fine chemicals such as enzymes, free polyssacharides, protein-bound polysaccharides, lectins, terpenes, antibiotics and alkaloids from either spent mushroom compost, mushroom stems or partially degraded mushroom fruiting bodies. This process will also contribute to health and environmental policies because it makes use of an agricultural waste material as raw material for novel processes.

### SUMMARY OF THE INVENTION

The aim of this process consists of simultaneous extraction and purification of fine chemicals (i.e enzymes, protein - bound polysaccharides, lectins, antibiotics, alkaloids and terpenes) from either spent mushroom compost, mushroom stems or partially degraded mushroom fruiting bodies. Several mushroom strains can be used for this purpose such as *Agaricus bisporus, Pleurotus ostreatus, Lentinula edodes, Cordyceps sinensis, Grifola frondosa, Ganoderma lucidium, Poria cocus, Polyporus umbelatus, Hericium erinaceus, Auricularia auricular* and *Coriolus versicolor.* These raw materials can be used for simultaneous extraction and purification of laccase, tyrosinase, pectinases, superoxide dismutase, xylanase, protease, cellulases, intracellular and extracellular protein-bound polysaccharides, lectins, antibiotics and terpenes. As far as enzymes, lectins and protein-bound polysaccharides are concerned, they can be separated and purified by gel filtration chromatography and immobilized metal affinity chromatography. On the other hand, several secondary metabolites including terpenes can be extracted either from the extracellular fluid or intracellular biomass by using suitable organic solvents such as methanol, acetone and ethyl acetate. Such secondary metabolites can be separated and purified by GC-MS and HPLC

### DESCRIPTION OF THE DRAWING

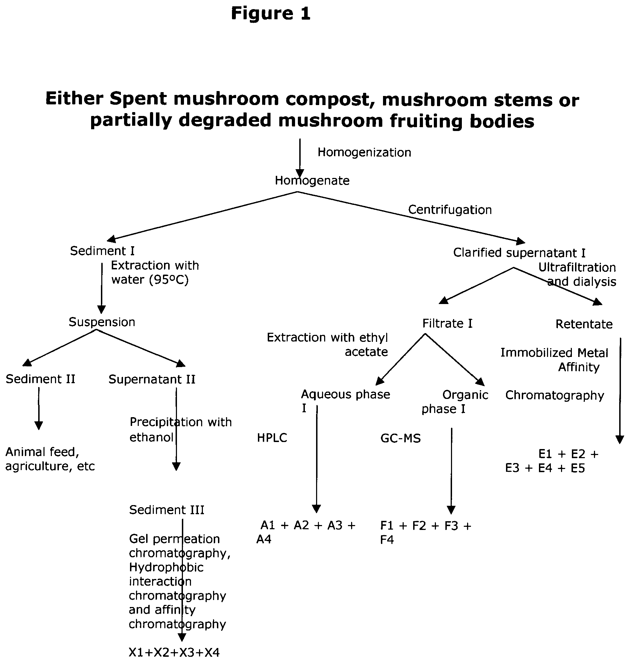

Fig. 1 is a scheme illustrating the simultaneous extraction and purification of fine chemicals from either spent mushroom compost, mushroom stems or partially degraded mushroom fruiting bodies.

### DETAILED DESCRIPTION OF THE INVENTION

The process for simultaneous extraction and purification of fine chemicals from wastes of mushroom production is presented in Figure 1.

### Example 1

100g (wet weight) of either spent mushroom compost, mushroom stems or degraded mushroom fruiting bodies was homogeneized with 3 volumes of 50 mM phosphate buffer pH 7.0 by using a Waring blendor for 15 min. at 4°C. The suspension was centrifuged at 5000 xg for 10 min. and the clear supernatant (Supernatant I) was concentrated by ultrafiltration with P10 membrane. The retentate was applied to a column packed with epoxy-activated Sepharose 6B- IDA- Cu(II) which was previously equilibrated with 20 mM phosphate buffer pH 7.0 containing 1M NaCl. The column was washed with the same buffer system and adsorbed proteins were eluted with a linear gradient of imidazole (0- 75 mM) in the same buffer system. The column fractions were analysed for protein, enzyme activity, anti-bacterial and anti-viral activities. The filtrate (I) was extracted with equal volume of ethyl acetate by partitioning in a separating funnel (solvent-solvent extraction) and the organic extract/organic phase (I) was evaporated to dryness *in vacuo.* The dried extract was reconstituted in minimal amount of 50% aqueous methanol and were purified by GC-MS and HPLC. The purified fractions were tested for anti-bacterial and anti-viral activities. The aqueous extract/aqueous phase (I) was applied to an HPLC column for separation of secondary metabolites. The analytical column used was a Nucleosil 100-7 C-18 (250 x 4.6 mm i.d.) with a guard column of the same material (CC 8/4 Nucleosil 100-5 C18). Methanol and water (1:1) were used as mobile phase at a flow rate of 1ml/min at 25°C.

The sediment (I) from Figure 1 was resuspended with one volume of water, heated at 100°C for 2 hours and the suspension was centrifuged at 5000xg for 5 min. The supernatant (II) was precipitated with four volumes of ethanol and the sediment (III) was redissolved in minimum volume of water. The free and protein-bound polysaccharides were separated and purified by gel-permeation chromatography on a column packed with Sephacryl HR-100 which was previously equilibrated with 20 mM phosphate buffer pH 7.0. Free and protein-bound polysacharides were eluted from the column with the same buffer system at a flow rate of 30 ml/h and column fractions were analysed by FTIR, UV-VIS and NMR as well as for anti-tumour, anti-viral, anti-fungal and anti-bacterial activities. The sediment (II) was used either as garden fertilizer or in animal feed.

### Example 2

100g (wet weight) of either spent mushroom compost, mushroom stems or degraded mushroom fruiting bodies was homogeneized with 4 volumes of 20 mM phosphate buffer pH 6.0 by using a Waring blendor for 10 min. at 4°C. The suspension was centrifuged at 5000 xg for 10 min. and the clear supernatant (Supernatant I) was concentrated by ultrafiltration with P10 membrane. The retentate was applied to a column packed with epoxy-activated Sepharose CL 6B- IDA- Cu(II) which was previously equilibrated with 20 mM phosphate buffer pH 6.0 containing 1M NaCl. The column was washed with the same buffer system and adsorbed proteins were eluted with a linear gradient of imidazole (0-50 mM) in the same buffer system. The column fractions were analysed for protein, enzyme activity, anti-bacterial and anti-viral activities. The filtrate (I) was extracted with equal volume of ethyl acetate by partitioning in a separating funnel (solvent-solvent extraction) and the organic extract (I) was evaporated to dryness *in vacuo.* The dried extract was reconstituted in minimal amount of 20% aqueous methanol and were purified by GC-MS and HPLC. The purified fractions were tested for anti-bacterial and anti-viral activities. The aqueous extract (I) was applied to an HPLC column for separation of secondary metabolites. The analytical column used was a Nucleosil 100-7 C-18 (250 x 4.6 mm i.d.) with a guard column of the same material (CC 8/4 Nucleosil 100-5 C18). Methanol and water (0.5:1) was used as mobile phase at a flow rate of 1ml/min at 25°C.

The sediment (I) from Figure 1 was resuspended with one volume of water, heated at 95°C for 3 hours and the suspension was centrifuged at 5000xg for 5 min. The supernatant (II) was precipitated with four volumes of ethanol and the sediment (III) was redissolved in minimum volume of water. The free and protein-bound polysaccharides were separated and purified by gel-permeation chromatography on Sephacryl HR-300 which was previously equilibrated with 20 mM phosphate buffer pH 6.0. Free and protein-bound polysacharides were eluted from the column with the same buffer system at a flow rate of 35 ml/h and column fractions were analysed by FTIR, UV-VIS and NMR as well as for anti-tumour, anti-viral, anti-fungal and anti-bacterial activities. The sediment (II) was used either as garden fertilizer or in animal feed.

### Methods

### 1. Preparation of chromatographic matrices

Epoxy-activated agarose gel containing 1,4-butanedioldiglycidyl ether as spacer arm was prepared as described in the literature. Under the conditions selected, the agarose matrix contained 30 µmole of epoxide groups/ml of sedimented gel. Subsequently, epoxy-activated agarose was reacted with iminodiacetic acid (IDA) as the chelating agent and the stationary phase thus obtained was thoroughly washed with water and kept at 4°C in 0.01% (w/v) sodium azide solution.

### 2. Enzyme assays

Total polygalacturonase or pectinase activity was assayed by incubating either the culture supernatant or column fractions, for 1 hour at 50°C, with 0.5 % (wt/vol.) polygalacturonic acid in 50 mM citrate buffer pH 4.8. The enzyme activity was determined from a calibration curve by using galacturonic acid as a standard. Reducing sugars in the reaction mixture were determined by the dinitrosalicylic acid method .One enzyme unit is defined as the amount of enzyme required for formation of 1 µmole of reducing sugars per minute at 50°C. Laccase (EC 1.10.3.2) was assayed by using o-dianisidine as a substrate. Assays were performed in 0.2M sodium acetate buffer at pH 4.5 containing 17 mM o-dianisidine and the oxidized product was read at 450nm as described in the literature; One unit of laccase activity was defined as the amount of enzyme required to oxidize 1 µmole substrate per min. Total xylanase (EC 3.2.1.8) activity was assayed by incubating the extract, for 1 hour at 50°C, with 0.5 % (w/v.) birchwood xylan in 0.05 M citrate buffer pH 4.8. The increase in reducing sugars in the reaction mixture was determined by the dinitrosalicylic acid method by using xylose as standard. All other enzyme assays were carried out as described in the literature.

### 3. Free and protein-bound polysaccharides

Free and protein-bound polysaccharides were assayed by phenol-sulphuric acid for total sugar content. Subsequently, these samples were analysed by using HPLC with a UV and RI detectors and a GPC column (Shodex) with a mobile phase containing deionised water at a flow rate of 1.0 ml/min at 25°C.

### 4. Protein assay

Total protein was determined by Bradford method by using BSA as a protein standard.

### 5. Biological activity of secondary metabolites and polysaccharides

Antibacterial Activity. The organic and aqueous extracts were studied for antibacterial activity against four bacteria: *Bacillus cereus, Bacillus megaterium, Sarcina lutea* and *Staphylococcus aureus.* The agar disc diffusion protocol was used for antibacterial assay. Sterile filter paper disc of 6 mm in diameter were loaded with 250 µg/disc and 150 µg/disc of the sample extract and were dried under laminar air flow cabinet. Standard antibiotic, streptomycin was used as a positive control. The loaded discs were placed in petri dish (90 mm in diameter) containing sterile nutrient agar medium inoculated with test microorganisms.

Assay of anti-proliferative activity on tumor cell lines. The anti-proliferative activity of the purified secondary metabolite was determined as follows. The cell lines L1210, M1, and HepG2 were purchased from ATCC which were maintained in Dulbecco's modified Eagle's medium (DMEM) supplemented with 10% fetal bovine serum (FBS), 100 mg/L streptomycin, and 100 IU/ml penicillin at 37 °C in a humidified atmosphere of 5% CO2. Cells in exponential growth phase were seeded into each well of a 96-well culture plate and incubated for 3 h before addition of the secondary metabolite. Incubation was carried out for another 48 h. Radioactive precursor was then added to each well and incubated for 6 h. The cultures were then harvested by a cell harvester. The incorporated radioactivity was determined by liquid scintillation counting.

Assay of secondary metabolite for antifungal activity. The assay for antifungal activity was performed by using sterile Petri dishes containing 10 ml potato dextrose agar. Sterile paper discs, 0.625 cm in diameter, were placed at a distance of 1 cm from the rim of the mycelial colony. An aliquot of the secondary metabolite was added to a disc. Incubation of the petri dish was carried out at 23 °C for 72 h until mycelial growth had enveloped peripheral disks containing the control and had formed crescents of inhibition around discs with antifungal samples. Three fungal species, *Fusarium oxysporum, Botrytis cinerea* and *Mycosphaerella arachidicola,* were examined in the assay. The antifungal protein red kidney bean lectin was used as a positive control.

Assay of inhibitory activity toward HIV reverse transcriptase. The assay for HIV reverse transcriptase inhibition activity was conducted in a nonradioactive ELISA kit from Boehringer-Mannheim. The inhibition assay was performed as described in the protocol included with the kit. The inhibitory activity of the secondary metabolite was calculated as percent inhibition as compared to a control without the secondary metabolite. *A*. *bisporus* lectin and red kidney bean lectin were used as positive controls.

6. Analytical techniques- Several physico-chemical techniques were used for identification of free and protein-bound polysaccharides as well as terpenes, alkaloids and antibiotics such as FTIR, UV-VIS, GC-MS and NMR.

### REFERENCES

Sanchez, C. (2004) Modern aspects of mushroom culture technology Appl Microbiol Biotechnol 64: 756-762.
Lau, K.L, Tsang, Y.Y, Chiu, S.W (2003) Use of spent mushroom compost to bioremediate PAH-contaminated samples. Chemosphere 52:1539-1546
Xawek V, Bhatt T, Cajthami T, Malachová K, Lednicka D (2003) Compost-mediated removal of polycyclic aromatic hydrocarbons from contaminated soil. Arch Environ Contam Toxicol. 44:336-342.
Gutiérrez R, Valle EMM, Galán MA. (2007) Immobilized Metal-Ion Affinity Chromatography: Status and Trends. Separation & Purification Reviews, 36:71-111.
Bailey MJ, Biely P, Poutanen K. (1992) Interlaboratory testing of methods for assay of xylanases activity. J Biotechnol 23:257-270.
Bradford MM (1976). A rapid and sensitive method for the quantitation of microgram quantities of protein utilizing the principle of protein-dye binding. Anal Biochem 72:248-54.
Pacheco V, Karmali A (1998) Chromatographic behaviour of glucose 1- and 2-oxidases from fungal strains on immobilized metal chelates. J Indust Microbiol Biotechnol 21, 57-64.
White CA, Kennedy JF. (1986) Oligosaccharides. In: Chaplin M, Kennedy JF. (eds) Carbohydrate analysis: a practical approach, IRL Press Limited, Oxford, UK.
Karmali, A.(2005) Differential chromatographic behaviour of some lignolytic enzymes from white-rot basidiomycetes on immobilized metal chelates" International J. Medicinal Mushrooms 3, 418.

## Claims

1. The complex scheme of unit operations shown in Fig.1.
for simultaneous extraction and purification of enzymes, lectins, free and protein- bound polysaccharides with triple helix structure of β-1,3-1,6-glucan and secondary metabolites including terpenes, alkaloids and antibiotics from either spent mushroom compost, mushroom stems or partially degraded mushroom fruiting bodies as follows:
Pyranose dehydrogenase, xylanase, pectinase, protease and laccase from spent mushroom compost of *Agaricus bisporus* defined as retentate in Fig. 1 are separated by immobilized metal affinity chromatography (IMAC) by using epoxy- activated Sepharose 6B- IDA- Cu(II) as chromatographic matrix and 20 mM phosphate buffer pH 6.0 or 7.0 containing 1M NaCl as mobile phase.
Tyrosinase, superoxide dismutase and protein-bound polysaccharides with triple helix structure of β-1,3-1,6--glucan from partially degraded mushroom fruiting bodies defined as retentate in Fig.1 are separated by immobilized metal affinity chromatography using epoxy- activated Sepharose 6B- IDA- Cu(II) as chromatographic matrix and 20 mM phosphate buffer pH 6.0 or 7.0 containing 1 M NaCl, as mobile phase.
Extracellular free and protein-bound polysaccharides with triple helix structure of β-1,3-1,6--glucan from spent mushroom compost defined as Sediment III in Fig.1 are separated by extraction, precipitation and gel-filtration chromatography by using Sephacryl S-300 HR-300 or 100 and 20 mM phosphate buffer pH 6.0 or 7.0, respectively, as chromatographic matrix and mobile phase, respectively.
Terpenes, alkaloids and antibiotics with biological activities were separated by extraction and HPLC from spent mushroom compost defined as aqueous phase I in Fig. 1 by Nucleosil 100-7 C-18 250 x 4.6 mm i.d. with a guard column of CC 8/4 Nucleosil 100-5 C18 and methanol and water (0.5:1), as chromatographic matrix and mobile phase, respectively.

2. A method according to claim 1 wherein the mushroom strains are *Agaricus bisporus, Pleurotus ostreatus, Lentinula edodes, Cordyceps sinensis, Grifola frondosa, Ganoderma lucidium, Poria cocus, Polyporus umbelatus, Hericium erinaceus, Auricularia auricular* and *Coriolus versicolor.*

## Patentansprüche

1. Das komplexe Schema der Einzeloperationen gezeigt in Fig. 1. zur gleichzeitigen Beseitigung und Aufreinigung von Enzymen, Lektinen, freien und proteingebundenen Polysacchariden mit Dreifachhelix-Struktur von ß-1,3-1,6-Glucane und sekundären Metaboliten ,einschließlich Terpenen, Alkaloiden und Antibiotika entweder aus verwendeten Pilzkomposten, Pilzstiele oder teils verdorbenen Pilzfruchtkörpern wie folgt:
Pyranosen dehydrogenase, Xylanasen, Pektinasen, Proteasen und Laccasen aus verwendeten Pilzkomposten von *Agaricus bisporus* definiert als Retentat in Fig. 1 sind durch Immobilisierte-Metallionen-Affinitätschromatographie (IMAC) getrennt bei Anwendung von Epoxy-aktivierter Sepharose 6B-IDA-Cu(II) als chromatographische Matrix und 20mM Phosphat-Puffer pH 6.0 oder 7.0, der 1M NaCl als mobile Phase enthält.
Tyrosinasen, Superoxid-Dismutasen und proteingebundene Polysacchariden mit Dreifachhelix-Struktur von ß-1,3-1,6-Glucane aus teils verdorbenen Pilzfruchtkörpern definiert als Retentat in Fig. 1 sind durch Immobilisierte-Metallionen-Affinitätschromatographie getrennt bei Anwendung von Epoxy-aktivierter Sepharose 6B-IDA-Cu(II) als chromatographische Matrix und 20mM Phosphat-Puffer pH 6.0 oder 7.0, der 1M NaCl als mobile Phase enthält.
Extrazellenfreie und proteingebundene Polysacchariden mit Dreifachhelix-Struktur von β-1,3-1,6-Glucane aus verwendeten Pilzkomposten definiert als Sediment III in Fig. 1 sind durch Beseitigungs-, Ausscheidungs- und Gel-Filtrationschromatographie getrennt bei Anwendung von Sephacryl S-300 HR-300 oder 100 und 20 mM Phosphat-Puffer pH 6.0 oder 7.0, entsprechend als chromatographische Matrix und mobile Phase, entsprechend.
Terpenen, Alkaloiden und Antibiotika mit biologischen Aktivitäten wurden getrennt durch Beseitigung und HPLC aus verwendeten Pilzkomposten definiert als wasserliche Phase I in Fig. 1 durch Nucleosil 100-7 C-18 250 x 4.6 mm, d.h. mit einer Vorsäule von CC 8/4 Nucleosil 100-5 C18 und Methanol und Wasser (0.5:1), als chromatographische Matrix und mobile Phase, entsprechend).

2. Eine Methode gemäß Anspruch 1, wobei die Pilzstämme *Agaricus bisporus, Pleurotus ostreatus, Lentinula edodes, Cordyceps sinensis, Grifola frondosa, Ganoderma lucidium, Poria cocus, Polyporus umbelatus, Hericium erinacaeus, Auricularia auricular* und *Coriolus versicolor* sind.

## Revendications

1. Schéma du complexe des opérations de l'unité présenté dans la Fig.1. pour l'extraction et la purification simultanées des enzymes, des lectines, des polysaccharides libres et liés aux protéines avec une structure en triple hélice de β-1,3-1,6-glucane et des métabolites secondaires, incluant des terpènes, des alcaloïdes et des antibiotiques tant de compost de champignon usé, que de pieds de champignons ou de corps de fructification de champignon partiellement dégradé, comme il suit :
le pyranose déshydrogénase, la xylanase, la pectinase, la protéase et la laccase de compost de champignon usé *d'Agaricus bisporus,* définis comme rétentat dans la Fig. 1 sont séparés par chromatographie d'affinité de métal immobilisé (IMAC) en utilisant du Sepharose 6B- IDA-Cu(II) activé en époxy comme matrice chromatographique et tampon de phosphate 20 mM de pH de 6.0 ou de 7.0, contenant 1M NaCl comme phase mobile.
La tyrosinase, la superoxyde dismutase et les polysaccharides liés aux protéines avec une structure en triple hélice de β-1,3-1,6-glucane de corps de fructification de champignon partiellement dégradé, définis comme rétentat dans la Fig. 1 sont séparés par chromatographie à d'affinité de métal immobilisé en utilisant du Sepharose 6B- IDA- Cu(II) activé en époxy comme matrice chromatographique et tampon de phosphate 20 mM de pH à 6.0 ou 7.0, contenant 1 M NaCI, comme phase mobile.
Les polysaccharides extracellulaires libres et liés aux protéines avec une structure en triple hélice de β-1,3-1,6 -glucane de compost de champignon usé, défini comme Sédiment III, dans la Fig. 1 sont séparés par extraction, précipitation et chromatographie de filtration au gel en utilisant la Séphacryl S-300 HR-300 ou 100 et le tampon de phosphate 20 mM de pH 6.0 ou 7.0 respectivement, comme matrice chromatographique et phase mobile, respectivement.
Les terpènes, alcaloïdes et antibiotiques avec des activités biologiques ont été séparés par extraction et HPLC depuis le compost de champignon usé, défini comme phase aqueuse I dans la Fig. 1 par Nucleosil 100-7 C-18 250 x 4.6 mm de diamètre interne, avec une colonne de garde de CC 8/4 Nucleosil 100-5 C18 et méthanol et eau (0.5:1), comme matrice chromatographique et phase mobile, respectivement).

2. Méthode selon la revendication 1 où les souches de champignons sont : *Agaricus bisporus, Pleurotus ostreatus, Lentinula edodes, Cordyceps sinensis, Grifola frondosa, Ganoderma lucidium, Poria cocus, Polyporus umbelatus, Hericium erinaceus, Auricularia auricular* et *Coriolus versicolor.*
